# EUROPEAN PATENT APPLICATION

(11) **EP 0 925 782 A2**
(43) Date of publication of application: **30.06.1999**
(21) Application number: 98403133.6
(22) Date of filing: 11.12.1998
(51) Int. Cl.: A61K 7/48, A61K 7/50

(54) **Cosmetics**

(30) Priority: 15.12.1997 JP 36253097
(71) Applicant: SOGO PHARMACEUTICAL COMPANY LIMITED, Chiyoda-ku Tokyo (JP)
(72) Inventor: Ohsumi, Tomoko, Kanagawa-ken (JP); Uchikuga, Saburoh, Yokohama-shi, Kanagawa-ken (JP)
(74) Representative: Clisci, Serge

(57) **Abstract**

The present invention relates to cosmetics of which the moisturizability is increased by containing glutaurine or its salt.

## Description

### Detailed Description of the Invention

### Field of the Invention

The present invention relates to cosmetics containing glutaurine or its salt.

More specifically, the present invention relates to cosmetics which increase the moisturizability and maintain a fresh skin for a long period of time by glutaurine or its salt.

### Prior Art

In order to increase a moisturizability of cosmetics, a moisturizer such as glycerin, propylene glycol, polyethylene glycol, sorbitol or the like has been often used so far. Problems To Be Solved by the Invention

A certain moisturizing effect was found in the conventional moisturizers. However, properties of each moisturizer, for example, a sticky touch, were undesirably provided in some cases, and a better moisturizer has been in demand.

### Brief Description of the Drawings

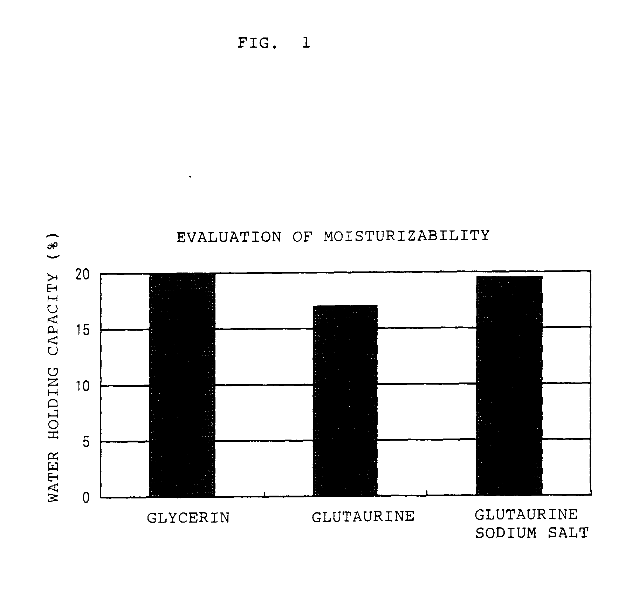

Figure 1 is a graph showing results of a test for a moisturizability of glutaurine or glutaurine sodium salt relative to glycerin.

### Means For Solving the Problems

Assiduous investigations have been made for finding a better moisturizer in cosmetics, and it has consequently been found in the present invention that glutaurine or its salt exhibits an excellent moisturizability and also desirable properties, and is appropriate as a moisturizer in cosmetics. 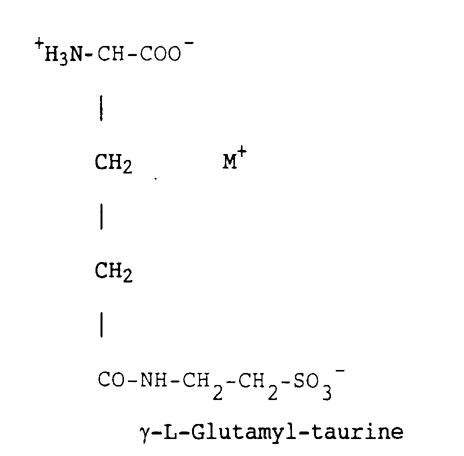 wherein M represents hydrogen, an alkali metal or a 1/2 alkaline earth metal.

Glutaurine or its salt used in the present invention is represented by the above-mentioned formula.

Glutaurine was first found in an extract of bovine. Its presence as a brain peptide was then identified, and the physiological activity like that of vitamin A has been reported.

Glutaurine is a substance which is easily synthesized (Synthesis, April 1992, pp. 353 - 354). Further, the acute toxicity of glutaurine is shown in terms of LD₅₀ > 1000 mg/kg (rat, p. o.).

In the present invention, glutaurine or its salt is added to cosmetics as such or through an appropriate medium. Glutaurine or its salt is dissolved or dispersed in a liquid preparation or a powder, and mixed with other materials to produce cosmetics, for example, a lotion, a washing foam, a cream and a pack.

The amount of glutaurine or its salt in cosmetics is between 0.001 and 30% by weight, preferably between 0.01 and 10% by weight, more preferably between 0.05 and 5% by weight.

In the cosmetics of the present invention, the moisturizability is increased with glutaurine or its salt. As other components used in the cosmetics, a surface active agent, an oil, a powder, an ultraviolet absorber, an antiseptic, a pigment, a flavor and the like can be incorporated as required. Examples

The present invention is illustrated by referring to the following Examples.

### (Test for a moisturizability of glutaurine or glutaurine sodium salt)

Approximately one gram of a sample was taken in a weighing bottle, and dried with diphosphorus pentoxide under reduced pressure for 4 hours. The resulting sample was used in the following test. The weight was accurately measured, and the sample was allowed to stand in a constant temperature and constant humidity box having a temperature of 20°C and a humidity of 75% for 7 days. Subsequently, the sample was moved in a constant temperature and constant humidity box having a temperature of 20°C and a humidity of 45%, and allowed to stand for 7 days. The moisturizability was evaluated from the change in weight. As a control sample, glycerin was used. The results are shown in Table 1 and Figure 1. The sample exhibited approximately the same moisturizability as glycerin, a typical moisturizer.

**Table 1**

| | Glycerin | Glutaurine | Glutaurine sodium salt |
|---|---|---|---|
| Water holding capacity (%) | 19.88 | 17.12 | 19.45 |

### Example 1

### (Lotion)

A lotion was prepared according to the following formulation.

| | weight % |
|---|---|
| 1. glutaurine | 3.0 |
| 2. propylene glycol | 4.0 |
| 3. oleyl alcohol | 0.1 |
| 4. polyoxyethylenesorbitan monolaurate | 1.0 |
| 5. polyoxyethylene lauryl ether | 0.5 |
| 6. ethanol | 10.0 |
| 7. flavor | 0.1 |
| 8. antiseptic | suitable amount |
| 9. purified water | balance |
| Total amount | 100.0 |

Components 1 and 2 were added to purified water, and dissolved therein at room temperature. Meanwhile, components 3, 4, 5, 7 and 8 were added to ethanol, and dissolved therein at room temperature. The resulting solution was added to the former purified water portion, and dissolved therein. Subsequently, the mixture was filtered to prepare a lotion.

### Example 2

### (cream)

A cream was prepared according to the following formulation.

| | weight % |
|---|---|
| 1. MGS-B | 1.2 |
| 2. BB-5 | 0.6 |
| 3. BB-20 | 1.2 |
| 4. Bees Wax Gold | 3.0 |
| 5. GM-18S | 1.0 |
| 6. bathyl alcohol | 18.0 |
| 7. CIO | 5.0 |
| 8. 1,3-BG | 0.1 |
| 9. methyl paraben | 0.1 |
| 10. propyl paraben | 0.1 |
| 11. sodium dehydroacetate | 0.1 |
| 12. glutaurine | 1.0 |
| 13. hypotaurine | 2.0 |
| 14. flavor | 0.1 |
| 15. purified water | balance |
| Total amount | 100.0 |

Components 1 to 10 were heat-dissolved at 85°C. Components 11 to 15 were heat-dissolved at 90°C, and the solution was mixed with former solution. The mixture was strongly stirred using a homomixer. The stirring was gently conducted from approximately 75°C, and stopped at from 30 to 35°C.

### Example 3

### (Lotion)

A lotion was prepared according to the following formulation.

| | weight % |
|---|---|
| 1. glutaurine sodium salt | 3.0 |
| 2. propylene glycol | 4.0 |
| 3. oleyl alcohol | 0.1 |
| 4. polyoxyethylenesorbitan monolaurate | 1.0 |
| 5. polyoxyethylene lauryl ether | 0.5 |
| 6. ethanol | 10.0 |
| 7. flavor | 0.1 |
| 8. antiseptic | suitable amount |
| 9. purified water | balance |
| total amount | 100.0 |

Components 1 and 2 are added to purified water, and dissolved therein at room temperature. Meanwhile, components 3, 4, 5, 7 and 8 were added to ethanol, and dissolved therein at room temperature. The resulting solution was added to the former purified water portion, and dissolved therein. Subsequently, the mixture was filtered to prepare a lotion.

### Example 4

### (Cream)

A cream was prepared according to the following formulation.

| | weight % |
|---|---|
| 1. MGS-B | 1.2 |
| 2. BB-5 | 0.6 |
| 3. BB-20 | 1.2 |
| 4. Bees Wax Gold | 3.0 |
| 5. GM-18S | 1.0 |
| 6. bathyl alcohol | 18.0 |
| 7. CIO | 5.0 |
| 8. 1,3-BG | 0.1 |
| 9. methyl paraben | 0.1 |
| 10. propyl paraben | 0.1 |
| 11. sodium dehydroacetate | 0.1 |
| 12. glutaurine sodium salt | 1.0 |
| 13. hypotaurine | 2.0 |
| 14. flavor | 0.1 |
| 15. purified water | balance |
| Total amount | 100.0 |

Components 1 to 10 were heat-dissolved at 85°C. Components 11 to 15 were heat-dissolved at 90°C, and the solution was mixed with former solution. The mixture was strongly stirred using a homomixer. The stirring was gently conducted from approximately 75°C, and stopped at from 30 to 35°C. Effects of the Invention

In the present invention, cosmetics containing glutaurine or glutaurine sodium salt have an excellent moisturizability. Accordingly, when the cosmetics are used, a feeling of quick drying disappears, and a moist skin feeling can be provided.

## Claims

1. Cosmetics containing glutaurine or its salt represented by the chemical formula 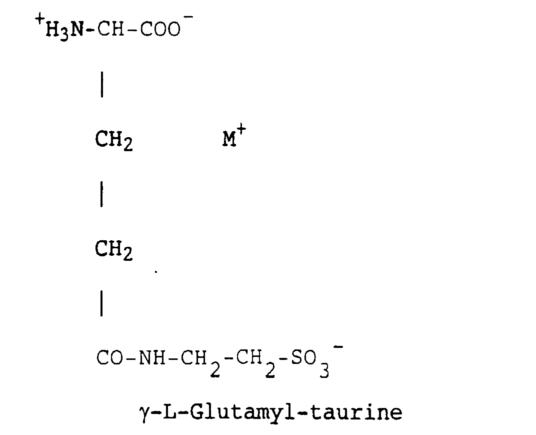 wherein M represents hydrogen, an alkali metal or a 1/2 alkaline earth metal.

2. Cosmetics of which the moisturizability is increased by containing glutaurine or its salt.
